# FASCICULE DE BREVET EUROPEEN

(11) **EP 2 061 893 B1**
(45) Date de publication et mention de la délivrance du brevet: **16.07.2014**
(21) Numéro de dépôt: 07823493.7
(22) Date de dépôt: 10.09.2007
(51) Int. Cl.: C12P 19/44, C12P 19/22, C12P 19/14, C12P 19/16, C07H 15/04, A23L 1/236

(54) **Procédé d'obtention d'un sirop à haute teneur en maltitol**
Verfahren zur Herstellung eines Sirups mit hohem Maltitolgehalt
Process for obtaining a syrup with a high maltitol content

(30) Priorité: 08.09.2006 FR 0607885
(43) Date de publication de la demande: 27.05.2009
(73) Titulaire: Syral, 67390 Marckolsheim (FR)
(72) Inventeur: SPINNER, Christophe, 67750 Scherwiller (FR); WAGNER, Anne, 67730 Chatenois (FR)
(74) Mandataire: Nuss, Laurent
(86) Numéro de dépôt international: PCT/FR2007/001457
(87) Numéro de publication internationale: WO 2008/029033

(56) Documents cités:
- EP-A2- 0 185 595
- EP-A2- 1 016 728
- WO-A-2004/003236
- WO-A-2005/014608
- US-A- 5 462 864
- US-A1- 2002 158 021
- NEBESNY E: "CARBOHYDRATE COMPOSITIONS AND MOLECULAR STRUCTURE OF DEXTRINS IN ENZYMATIC HIGH MALTOSE SYRUPS" STARCH, vol. 42, no. 11, 1990, pages 437-444, XP002467238 ISSN: 0038-9056

## Description

La présente invention concerne un procédé de fabrication d'un sirop très riche en maltitol.

On connaît déjà de nombreux procédés permettant d'obtenir des sirops riches en maltose.

En général, les procédés connus comportent essentiellement au moins une étape de liquéfaction enzymatique d'un lait d'amidon suivie d'un certain nombres d'étapes de saccharification, également enzymatiques, une étape d'hydrogénation classique du maltose obtenu en maltitol. Les procédés connus présentent cependant plusieurs inconvénients, notamment du fait que certains parmi eux nécessitent en outre de nombreuses et fastidieuses étapes supplémentaires de séparation ou de purification qui diminuent les rendements et augmentent la complexité des opérations et celui des coûts d'exploitation.

Ainsi, la mise en oeuvre d'étapes successives de saccharification utilisant des enzymes spécifiques permet d'obtenir un sirop présentant une teneur en maltose supérieure à 87 % en poids (voir à ce sujet EP 0 905 256). L'utilisation d'une α-amylase maltogénique entraîne toutefois une concentration finale en glucose élevée (de plus de 6 % en poids) et entraîne, de ce fait, la nécessité d'une étape supplémentaire de transformation (oxydation du glucose en acide gluconique) suivie d'une étape de purification (désacidification sur résines échangeuses d'ions).

D'autres procédés classiques combinant l'utilisation d'enzymes spécifiques et de tamisages moléculaires (EP 1 016 728 ; US 5 462 864) permettent d'obtenir un sirop de maltose de pureté insuffisante qui, après hydrogénation catalytique, nécessite encore des étapes supplémentaires d'enrichissement comme la cristallisation afin d'obtenir un produit commercialement intéressant, par exemple contenant plus de 99,0 % de maltitol en poids.

Le document US 4 675 293 décrit l'utilisation combinée, lors de l'étape de saccharification, de β-amylase et d'α-amylase fongique suivie d'une hydrogénation catalytique. Dans ces conditions, un sirop de maltose avec une concentration en maltose comprise entre 60 % et 80 % en poids est obtenu. Une fois encore la teneur en glucose du sirop riche en maltose, respectivement la teneur en sorbitol (12 % à 14 % en poids) du sirop de maltitol obtenu est élevée et nécessiterait la mise en oeuvre de plusieurs étapes de purification non avantageuses économiquement dans le cadre de l'obtention d'un sirop de maltitol permettant sa cristallisation ou son séchage.

De même, le document EP 0 185 595 décrit la nécessité d'une purification d'un sirop de maltitol dont la composition serait proche de celle d'un sirop décrit dans le brevet US 4 675 293 ne permettant pas, malgré une étape de chromatographie, d'obtenir une pureté finale en maltitol suffisamment élevée.

De la même manière, l'utilisation d'une étape de chromatographie coûteuse en amont de l'étape d'hydrogénation (comme décrit dans le document WO 2005/014608), tout comme un tamisage moléculaire membranaire (décrit dans le document EP 1 354 067) qui permettent l'obtention de sirops très riches en maltose, semblent s'avérer inutiles au vu des coproduits générés lors de l'étape d'hydrogénation (hexitols par exemple) et nécessitent une étape de purification ultérieure supplémentaire.

Les procédés de fabrication de sirops de maltitol décrits dans les dépôts EP 0 185 595 ou US 4 675 293 qui prévoient un étape de saccharification d'un sirop à teneur intermédiaire en maltose, suivie d'une étape d'hydrogénation et d'une étape de séparation par chromatographie sont encore trop complexes et ne permettent pas d'atteindre ensuite un niveau de pureté supérieur à 98 % en poids (en masse) de maltitol sans une étape supplémentaire de cristallisation.

La présente invention a notamment pour but de pallier les inconvénients précités.

A cet effet, l'invention a pour objet un procédé d'obtention d'un sirop à haute teneur en maltitol dont le nombre et/ou la complexité des étapes unitaires soit réduite afin d'obtenir, d'une manière simple et peu coûteuse, un sirop d'une richesse en maltitol équivalente à celle obtenue par cristallisation et répondant à la monographie de la pharmacopée européenne (version 5.6).

Le procédé selon la présente invention est défini par la revendication 1.

De manière surprenante et contrairement aux procédés décrits dans les documents cités ci-dessus, le procédé selon l'invention permet l'obtention, et ceci lors d'une seule étape de saccharification, d'un sirop suffisamment riche en maltose et à la fois pauvre en glucose afin qu'il soit hydrogéné sans étape préalable de purification poussée telle qu'une étape de tamisage moléculaire ou de chromatographie. L'éventuelle filtration de l'étape c) ne constitue qu'une étape de filtrage (physique) grossier des résidus insolubles et ne peut être assimilée à une étape de séparation (chimique) des différents constituants moléculaires.

L'invention sera mieux comprise, grâce à la description ci-après, qui se rapporte à un mode de réalisation préféré, donné à titre d'exemple non limitatif.

La présente invention a donc pour objet un procédé d'obtention d'un sirop à haute teneur en maltitol caractérisé en qu'il comprend les étapes consistant à :
a) effectuer une liquéfaction d'un lait d'amidon avec une α-amylase pour obtenir un amidon liquéfié présentant un DE compris entre 5 et 10 (bornes incluses) ;
b) effectuer une unique étape de saccharification du lait d'amidon liquéfié obtenu à l'étape a) en présence simultanée d'au moins une β-amylase et d'au moins une enzyme débranchante comprenant une pullulanase et optionnellement aussi une isoamylase jusqu'à l'obtention d'une teneur en glucose de l'hydrolysat inférieure à 5 % en poids et d'une teneur en maltose de l'hydrolysat supérieure à 80 % en poids, le ratio de β-amylase/pullulanase étant compris entre 0,5 et 2 ;
c) effectuer après éventuelle filtration, déminéralisation et/ou concentration du sirop liquide tel qu'il a été obtenu à l'étape b), une hydrogénation catalytique pour obtenir un sirop riche en maltitol.

De manière encore plus avantageuse, la teneur en glucose de l'hydrolysat à la fin de l'étape b) est inférieure à 1 % en poids.

Ceci a pour effet de permettre, d'une part, d'obtenir un sirop de maltitol dont la teneur en sorbitol est faible, ce qui améliore très significativement la productivité et le rendement des étapes de purification menant à l'obtention d'un maltitol de haute pureté. D'autre part, le sirop à faible teneur en sorbitol obtenu peut être directement commercialisé en l'état pour des applications alimentaires, notamment dans la biscuiterie ou la confiserie.

Selon une variante, on peut effectuer la saccharification unique du lait d'amidon de l'étape b) en présence simultanée d'une α-amylase maltogénique supplémentaire. Ceci permet notamment d'accroître le rendement de la conversion enzymatique en augmentant la quantité de substrat disponible pour la β-amylase mais présente l'inconvénient d'augmenter la teneur en glucose.

De façon essentielle, le procédé selon la présente invention est en outre caractérisé en ce qu'on effectue, sur le produit obtenu à l'étape c), un premier fractionnement chromatographique jusqu'à l'obtention d'un sirop liquide présentant une concentration en maltitol supérieure à 90,0 %, de préférence supérieure à 91,0 % en poids de matière sèche, puis un second fractionnement chromatographique sur le sirop liquide pré-purifié jusqu'à l'obtention d'un sirop liquide présentant une concentration en maltitol supérieure à 99,0 %. Cette voie permet d'obtenir de très hautes teneurs en maltitol dans le sirop final.

Comme précédemment évoqué, le sirop riche en maltitol issu de l'étape d'hydrogénation subit une purification en 2 étapes successives, avantageuses économiquement et dépourvues de risque de contamination bactériologique, permettant d'obtenir un sirop d'une richesse en maltitol suffisante pour qu'il soit déshydraté et utilisable dans des applications diverses comme la confiserie et le chocolat.

Selon une autre caractéristique, le sirop riche en maltose obtenu à l'étape b) peut encore avantageusement subir un traitement thermique complémentaire en présence d'une α-amylase lui assurant une bonne stabilité en solution.

Le principe général de l'étape du procédé décrite en a) est connu en soi par l'homme du métier et consiste à liquéfier un lait d'amidon, de 20 à 50 % de matière sèche, préférentiellement de 25 à 35 %, issu soit de blé, de pomme de terre ou de tout autre amidon végétal purifié.

La liquéfaction est conduite de façon classique suivant un procédé enzymatique en 2 étapes :
- la première étape consiste en un chauffage à une température comprise entre environ 105 °C et 110 °C du lait d'amidon en présence d'α-amylase thermostable sur une durée courte (par exemple de 5 à 10 mn).
- la deuxième étape consiste à maintenir le lait d'amidon traité thermiquement entre 95 °C et 100 °C durant 1h 30 à 2h.

Les conditions de pH, de matière sèche et de température sont propres au type d'enzyme utilisée et choisies afin d'obtenir un indice de DE ou « DE » (Dextrose Equivalent) compris entre 5 et 10, préférentiellement entre 6 et 9. On pourra éventuellement soumettre le lait d'amidon liquéfié à une inhibition thermique en sortie de liquéfaction selon la pratique courante bien connue de l'homme du métier.

L'amidon liquéfié est ensuite saccharifié à l'aide d'un mélange enzymatique contenant une enzyme ayant une activité spécifiquement maltogénique (β-amylase) et une enzyme ayant une activité débranchante (pullulanase ou isoamylase) hydrolysant spécifiquement les liaisons α-1,6 du lait d'amidon, ce qui permet, d'une part de réduire sensiblement la teneur en polymères de haut poids moléculaire et d'accélérer la cinétique de conversion en maltose.

La proportion respective de chaque enzyme est déterminée en fonction du spectre final à atteindre en maltose et en fonction de la teneur finale en polymères de glucose de Degré de Polymérisation (DP) supérieur à 11.

Dans le cadre de l'obtention d'un sirop de maltose conforme à l'invention, le ratio β-amylase/pullulanase est compris entre 0,5 et 2.

Les conditions de pH et température de cette étape de saccharification sont celles usuellement appliquées par la profession lors de l'utilisation connue de ces enzymes.

L'étape unique de saccharification dure généralement entre 15 et 40 heures, plus préférentiellement entre 20 et 30 heures et dans ces conditions on obtient un sirop dont la teneur en maltose est d'au moins 80 % en poids et, de préférence, aux environs de 85 % en poids.

Dans le même temps, la teneur en glucose de cet hydrolysat est inférieure à 10 % en poids, de préférence inférieure à 5 % et idéalement inférieure à 1 % en poids de glucose.

L'hydrolysat issu de cette étape b) de saccharification unique peut être soumis quelques secondes à un nouveau traitement thermique en présence ou non d'une α-amylase particulière ayant des propriétés déviscosifiantes.

De manière préférée, le sirop riche en maltose subit ensuite une étape de filtration (filtre à précouche par exemple) de manière à supprimer les résidus insolubles puis est déminéralisé et concentré.

L'hydrolysat ainsi obtenu est alors soumis à une étape d'hydrogénation catalytique en utilisant indifféremment comme catalyseur du nickel de Raney ou un catalyseur à base de ruthénium, ce dans les conditions habituelles connues de l'homme du métier.

L'hydrogénation catalytique est menée de manière connue en soi, à savoir de façon discontinue (procédé dit par lots ou en « batch »), en ajustant la matière sèche de l'hydrolysat entre 30 et 60 %, avec une pression d'hydrogène habituellement comprise entre 30 et 640 bars, une température de réaction comprise entre 100 °C et 150 °C et en utilisant une quantité de catalyseur allant de 1 à 10 % en poids par rapport à la charge à hydrolyser. Afin d'éviter l'apparition de sous-produits indésirables, le pH de la réaction est maintenu à une valeur supérieure à 7,0 par adjonction d'auxiliaires comme la soude ou le carbonate de soude.

La réaction d'hydrogénation est stoppée lorsque la teneur en sucres résiduels de la solution à traiter est inférieure à 0,5 % et de préférence inférieure à 0,3 % en poids. Le rédacteur est alors refroidi, le catalyseur récupéré par filtration et la solution de maltitol déminéralisée de façon classique.

Dans ces conditions, la solution obtenue contient au moins 75 % de maltitol en poids et plus préférentiellement plus de 77 % de maltitol en poids. La teneur en sorbitol du produit ainsi obtenu est quant à elle inférieure à 12 % en poids, de préférence inférieure à 5 % en poids.

Selon l'une des variantes conforme à l'invention, le sirop de maltitol obtenu est amené à une quantité de matière sèche comprise entre 30 % et 60 %.

Alternativement et conformément à l'invention, la fraction enrichie en maltitol peut être soumise, après ajustement de la matière sèche à environ 60 % par évaporation, à une étape de chromatographie mixte d'exclusion stérique et d'échange ionique sur un principe de séparation à 3 composants. La fraction liquide enrichie contient dans ce cas plus de 99,0 % de maltitol en poids avec un taux de recouvrement (rendement) supérieur à 97 %.

Ce sirop de maltitol peut subir une évaporation et être utilisable directement en remplacement de maltitol cristallisé conventionnel qui doit être dissolu avant utilisation dans des applications alimentaires comme par exemple la confiserie, les crèmes glacées ou encore la viennoiserie pâtisserie.

On peut également obtenir un produit de pureté en maltitol supérieure à 99,0 % en poids et de préférence supérieure à 99,5 %, en utilisant deux étapes successives de chromatographie d'exclusion ionique, à savoir :
- une première étape consiste à alimenter une colonne chromatographique constituée de 4 chambres en série, à l'aide de la solution de maltitol décrite plus haut : on obtient dans ces conditions, une fraction enrichie contenant environ 95 % de maltitol en poids avec un taux de recouvrement (rendement) supérieur à 87 %,
- une deuxième séparation chromatographique (3 composants), la fraction liquide enrichie contient alors au moins 99,5 % de maltitol en poids avec un taux de recouvrement du maltitol supérieur à 92 %.

L'invention sera maintenant décrite plus en détail à l'aide des exemples non limitatifs suivants :

### Exemple 1 :

Un lait d'amidon de maïs ayant une matière sèche de 35 % en poids est liquéfié de manière conventionnelle à l'aide d'une α-amylase thermostable (par exemple connue sous la dénomination « Thermamyl » commercialisée par la société Novozymes) en utilisant un dosage et des conditions de pH permettant l'obtention d'un DE compris entre 6 et 9.

L'activité enzymatique résiduelle est inhibée thermiquement à une température strictement supérieure à 135 °C pendant quelques secondes puis le lait d'amidon liquéfié est refroidi à une température inférieure à 60 °C, sa matière sèche ajustée à environ 30 % en poids et le pH amené à une valeur comprise entre 5 et 5,5.

On ajoute ensuite simultanément une β-amylase (par exemple connue sous la dénomination « Spezyme BBA », commercialisée par la société Genencor) et une pullulanase (« Promozyme D » commercialisée par la société Novozymes), à des doses respectives comprises entre 0,1 % et 0,2 % en poids qui permettent d'atteindre une teneur en maltose supérieure à 80 % en poids en moins de 40 heures.

Dans ces conditions, le spectre final de l'hydrolysat après 35 heures de saccharification est de (tous les % sont donnés en poids):
a) Lorsque le ratio pullulanase/β-amylase est de 0,7 :
   glucose : 0,5 %, maltose : 81 %, DP3 : 10 %, DP4 : 2 % et DP>5 : 6,5 %
   DP: degré de polymérisation des polymères présents
b) Lorsque le ratio pullulanase/β-amylase est de 1,3 :
   glucose : 0,5 %, maltose : 84 %, DP3 : 9 %, DP4 : 1 % et DP>5: 5,5 %
   DP: degré de polymérisation des polymères présents

L'hydrolysat subit ensuite une étape de traitement thermique à 130 °C en présence d'une α-amylase à un dosage 0,01 % en poids (par exemple, connue sous la dénomination « Thermamyl Supra », de la société Novozyme) puis est filtré, décoloré, déminéralisé et évaporé selon les techniques utilisées classiquement dans ce type de procédé.

### Exemple 2 :

Un lait d'amidon de maïs ayant une matière sèche de 35 % en poids est liquéfié de manière conventionnelle à l'aide d'une α-amylase thermostable (par exemple connue sous la dénomination « Thermamyl » commercialisée par la société Novozymes) en utilisant un dosage et des conditions de pH permettant l'obtention d'un DE compris entre 6 et 9.

L'activité enzymatique résiduelle est inhibée thermiquement à une température strictement supérieure à 135 °C pendant quelques secondes puis le lait d'amidon liquéfié est refroidi à une température inférieure à 60 °C, sa matière sèche ajustée à environ 20 % en poids et le pH amené à une valeur comprise entre 5 et 5,5.

On ajoute ensuite simultanément une β-amylase (par exemple connue sous la dénomination « Spezyme BBA » commercialisée par la société Genencor), une α-amylase (« Maltogénase 240L de Novozymes, par exemple) et une pullulanase (« Promozyme D » commercialisée par la société Novozymes), à des doses respectives de 0,15 %, 0,05 % et de 0,1 % en poids qui permettent d'atteindre à une température de réaction de 60 °C en 30 heures le spectre glucidique suivant (tous les % sont donnés en poids):
Dextrose ; 6 %, maltose : 77 %, DP3 : 10 %, DP>3 : 8 %
DP: degré de polymérisation des polymère présents

Les hydrolysals de maltose dont l'obtention est décrite dans les exemples 1 et 2 sont soumis à une étape d'hydrogénation catalytique dont les conditions sont les suivantes :
- la matière sèche de l'hydrolysat est ajustée entre 40 % et 50 % en poids,
- la quantité de catalyseur est comprise entre 2 % et 5 % en poids,
- la pression d'hydrogène est maintenue entre 20 et 40 bars, et
- la température de réaction est comprise entre 100 °C et 130 °C.

Dans ces conditions, la réaction est stoppée lorsque le taux de sucres réducteurs est inférieur à 0,3 % en poids. On obtient alors par analyse HPLC la composition suivante :

| % en poids | Exemple 1(a) | Exemple 1(b) | Exemple 2 |
|---|---|---|---|
| Sorbitol | 2 | 2 | 6 |
| Maltitol | 77 | 80 | 75 |
| Maltotriitol | 12 | 10 | 10 |
| Autres polyols | 9 | 8 | 9 |

Un sirop de maltitol obtenu par hydrogénation du sirop décrit dans l'exemple 1(a) est purifié puis évaporé à une matière sèche de 60 % en poids.

On utilise ce sirop pour alimenter une colonne chromatographique séparant 2 constituants (type SMB améliorée) à quatre chambres en série à une température de 80 °C.

Dans ces conditions et en utilisant un rapport volumique eau/sirop compris entre 2 et 4, on obtient une fraction enrichie en maltitol à environ 40 % de matière sèche de composition suivante :
Sorbitol : 6 %
Maltitol : 93 %
Maltotriitol : 0,5 %
Autres polyols : 0,5 %

Le taux de recouvrement en maltitol est supérieur à 87 %.

La fraction (liquide) enrichie en maltitol obtenue ci-dessus est ensuite évaporée après purification pour obtenir un taux de matière sèche de 60 % en poids et alimente une colonne de chromatographie spécifique permettant d'isoler 3 composants. La colonne est alimentée à 80 °C en utilisant un rapport volumique eau/sirop compris entre 4 et 6.

On obtient alors une fraction (liquide) enrichie en maltitol à environ 35 % en poids de matière sèche dont la composition est la suivante :
Sorbitol : 0,5 %
Maltitol : 99,3 %
Maltotriitol : 0,2 %
Autres polyols : 0 %

Le taux de recouvrement en maltitol est supérieur à 92 %.

Les sirops très riches en maltitol obtenus finalement dans les exemples 1 et 2 peuvent être soumis directement à un procédé de séchage permettant d'obtenir une poudre de maltitol conforme à la monographie de la pharmacopée européenne sans nécessiter d'étape supplémentaire de purification telle qu'une étape de cristallisation.

## Revendications

1. Procédé d'obtention d'un sirop liquide à haute teneur en maltitol présentant une concentration en maltitol supérieure à 99,0 % caractérisé en qu'il comprend les étapes consistant à :
a) effectuer une liquéfaction d'un lait d'amidon avec une α-amylase pour obtenir un amidon liquéfié présentant un DE compris entre 5 et 10 (bornes incluses) ;
b) effectuer une unique étape de saccharification du lait d'amidon liquéfié obtenu à l'étape a) en présence simultanée d'au moins une β-amylase et d'au moins une enzyme débranchante comprenant une pullulanase jusqu'à l'obtention d'une teneur en glucose de l'hydrolysat inférieure à 5 % en poids et d'une teneur en maltose de l'hydrolysat supérieure à 80 % en poids, le ratio de β-amylase / pullulanase étant compris entre 0,5 et 2 ;
c) effectuer après éventuelle filtration, déminéralisation et/ou concentration du sirop liquide tel qu'il a été obtenu à l'étape b), une hydrogénation catalytique pour obtenir un sirop riche en maltitol ;
d) effectuer, sur le produit obtenu à l'étape c), un premier fractionnement chromatographique jusqu'à l'obtention d'un sirop liquide présentant une concentration en maltitol supérieure à 90,0 % puis un second fractionnement chromatographique sur le sirop liquide pré-purifié jusqu'à l'obtention d'un sirop liquide présentant une concentration en maltitol supérieure à 99,0 %.

2. Procédé selon la revendication 1, **caractérisé en ce qu'**on effectue, sur le produit obtenu à l'étape c), un premier fractionnement chromatographique jusqu'à l'obtention d'un sirop liquide présentant une concentration en maltitol supérieure à 91,0 % puis un second fractionnement chromatographique sur le sirop liquide pré-purifié jusqu'à l'obtention d'un sirop liquide présentant une concentration en maltitol supérieure à 99,0 %.

3. Procédé selon la revendication 1 ou 2, **caractérisé en ce que** la teneur en glucose de l'hydrolysat à la fin de l'étape b) est inférieure à 1 % en poids.

4. Procédé selon l'une quelconque des revendications précédentes dans lequel l'enzyme débranchante comprend une pullulanase et une isoamylase.

## Patentansprüche

1. Verfahren zur Herstellung eines Flüssigsirups mit hohem Maltitgehalt mit einer Maltitkonzentration von über 99,0%, **dadurch gekennzeichnet, dass** es die Schritte umfasst, die aus folgenden bestehen:
a) Durchführen einer Stärkemilchverflüssigung mit einer α-Amylase, wodurch man zu einer verflüssigten Stärke mit einem DE-Wert zwischen (einschließlich) 5 und 10 gelangt;
b) Durchführen eines einzigen Verzuckerungsschritts der in Schritt a) erhaltenen verflüssigten Stärkemilch in Gegenwart von gleichzeitig mindestens einer β-Amylase und mindestens einem Entzweigungsenzym umfassend eine Pullulanase, bis man zu einem Glucosegehalt des Hydrolysats von weniger als 5 Gew.-% und einem Maltosegehalt des Hydrolysats von über 80 Gew.-% gelangt, wobei das β-Amylase/Pullulanase-Verhältnis zwischen 0,5 und 2 beträgt;
c) gegebenenfalls nach Filtration, Entmineralisierung und/oder Konzentration des Flüssigsirups, wie er in Schritt b) anfällt, Durchführen einer katalytischen Hydrierung, wodurch man zu einem Sirup mit hohem Maltitgehalt gelangt;
d) Durchführen einer ersten chromatographischen Fraktionierung mit dem in Schritt c) anfallenden Produkt, bis man zu einem Flüssigsirup mit einer Maltitkonzentration von über 90,0% gelangt, und anschließend Durchführen einer zweiten chromatographischen Fraktionierung mit dem vorgereinigten Flüssigsirup, bis man zu einem Flüssigsirup mit einer Maltitkonzentration von über 99,0% gelangt.

2. Verfahren nach Anspruch 1, **dadurch gekennzeichnet, dass** man mit dem in Schritt c) anfallenden Produkt eine erste chromatographische Fraktionierung durchführt, bis man zu einem Flüssigsirup mit einer Maltitkonzentration von über 91,0% gelangt, und anschließendes Durchführen einer zweiten chromatographischen Fraktionierung mit dem vorgereinigten Flüssigsirup, bis man zu einem Flüssigsirup mit einer Maltitkonzentration von über 99,0% gelangt.

3. Verfahren nach Anspruch 1 oder 2, **dadurch gekennzeichnet, dass** der Glucosegehalt des Hydrolysats am Ende von Schritt b) unter 1 Gew.-% beträgt.

4. Verfahren nach einem der vorhergehenden Ansprüche, wobei das Entzweigungsenzym eine Pullulanase und eine Isoamylase umfasst.

## Claims

1. Process for obtaining a liquid syrup with a high maltitol content having a maltitol concentration greater than 99.0%, **characterized in that** it comprises the steps consisting in:
a) carrying out a liquefaction of a starch milk with an α-amylase so as to obtain a liquefied starch having a DE of between 5 and 10 (limits included);
b) carrying out a single step of saccharification of the liquefied starch milk obtained in step a) in the simultaneous presence of at least one β-amylase and of at least one debranching enzyme comprising a pullulanase until a glucose content of the hydrolysate of less than 5% by weight and a maltose content of the hydrolysate of greater than 80% by weight are obtained, the β-amylase/pullulanase ratio being between 0.5 and 2;
c) after optional filtration, demineralization and/or concentration of the liquid syrup such as was obtained in step b), carrying out a catalytic hydrogenation so as to obtain a maltitol-rich syrup;
d) carrying out, on the product obtained in step c), a first chromatographic fractionation until a liquid syrup having a maltitol concentration of greater than 90.0% is obtained and then carrying out a second chromatographic fractionation on the pre-purified liquid syrup until a liquid syrup having a maltitol concentration of greater than 99.0% is obtained.

2. Process according to Claim 1, **characterized in that** a first chromatographic fractionation is carried out, on the product obtained in step c), until a liquid syrup having a maltitol concentration of greater than 91.0% is obtained and then a second chromatographic fractionation is carried out on the pre-purified liquid syrup until a liquid syrup having a maltitol concentration of greater than 99.0% is obtained.

3. Process according to Claim 1 or 2, **characterized in that** the glucose content of the hydrolysate at the end of step b) is less than 1% by weight.

4. Process according to any one of the preceding claims, in which the debranching enzyme comprises a pullulanase and an isoamylase.
